# EUROPEAN PATENT APPLICATION

(11) **EP 4 302 802 A1**
(43) Date of publication of application: **10.01.2024**
(21) Application number: 23183955.6
(22) Date of filing: 06.07.2023
(51) Int. Cl.: A61M 5/142, A61M 5/168, A61M 5/172, G01L 7/08

(54) **SYSTEM AND METHOD FOR DETECTING OCCLUSIONS IN A FLUID PATH**

(30) Priority: 07.07.2022 US 202263359010 P
(71) Applicant: Insulet Corporation, Acton MA 01720 (US)
(72) Inventor: CAUSEY, James, Simi Valley (US); D'ARCO, John, Wilmington (US)
(74) Representative: Peterreins Schley

(57) **Abstract**

Disclosed herein is a system for detecting and handling occlusions in the fluid path of the drug delivery device. The system includes a sensor disposed in-line with the fluid path for determining a change in pressure within the fluid path. The system further includes a machine learning model for analyzing readings from the sensor to detect and classify an occlusion in the fluid path based on the time series of readings from the sensor indicating a change in pressure. The system further takes remedial action to either compensate for the occlusion, clear the occlusion, or inform the user of the existence of the occlusion and recommends a course of action to the user.

## Description

### BACKGROUND

Many conventional automated drug delivery (ADD) systems are well known, including, for example, wearable drug delivery devices. The drug delivery device can be designed to deliver any type of liquid drug to a user. In specific embodiments, the drug delivery device can be, for example, an OmniPod^{®} drug delivery device manufactured by Insulet Corporation of Acton, Massachusetts. The drug delivery device can be a drug delivery device such as those described in U.S. Pat. No. 7,303,549, U.S. Pat. No. 7,137,964, or U.S. Pat. No. 6,740,059, each of which is incorporated herein by reference in its entirety.

Such drug delivery devices typically include a positive displacement pumping mechanism to force a liquid drug from a reservoir through a fluid path to the patient. The fluid path typically comprises a needle or conduit coupled at one end to the reservoir. The other end of the fluid path is positioned directly in the patient or may be coupled to a cannula which is inserted under the skin of the patient for delivery of the liquid drug. The cannula may be inserted via a needle mechanism wherein a needle/cannula combination is forced by an actuator into the skin of the user and thereafter the needle is withdrawn, leaving the cannula in place. The cannula may have one or more ports defined on a distal end thereof through which the liquid drug is dispensed.

It is not uncommon for the one or more ports to become fully or partially clogged or occluded. The occlusions may be caused, for example, by body fluids aggregating near the distal end of the cannula or needle, or insulin aggregates formed as the insulin denatures, and which accumulate in the fluid path. Most commonly, occlusions occur at or near the distal end of the cannula and may clog one or more ports. Less commonly, occlusions could occur at other points along the fluid path. The occlusions may reduce the amount of the liquid drug that can be dispensed or may potentially preclude dispensing of any of the liquid drug.

For continuous subcutaneous insulin infusion, a reduction in the insulin delivery due to a fluid occlusion can cause harm in people managing T1 or T2 diabetes. The venous glucose of a diabetic can change at a rate of up to about 2 mg/dL/minute. In the absence of a steady basal delivery of insulin, the patient may quickly become hyperglycemic, and may become so while thinking that insulin is being delivered properly, but which in fact is not due to a full or partial occlusion.

Thus, it would be desirable to be able to detect when occlusions occur that reduce or stop the delivery of insulin so as to enable the drug delivery system to take remedial action, or alternatively, warn the user of a potentially dangerous situation, so that the user can take remedial action.

### SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

In one embodiment of the described subject matter, disclosed herein is a sensor for detecting changes in pressure in the fluid path. The sensor is disposed in-line with the fluid path and can output a time series of sensor readings indicative of changes of pressure withing the fluid path caused by the pumping of the liquid drug into the fluid path by the pumping mechanism and/or by occlusions in the fluid path.

In additional embodiments of the described subject matter, a machine learning model may take as input the readings produced by the sensor to detect and classify occlusions within the fluid path, based on changes in the fluid path pressure profile derived from the time series of sensor readings. The machine learning model may associate pressure profiles detected in the fluid path with normal dosing patterns and may apply multivariate techniques to identify an occlusion.

### BRIEF DESCRIPTION OF THE DRAWINGS

In the drawings, like reference characters generally refer to the same parts throughout the different views. In the following description, various embodiments of the presently described subject matter are described with reference to the following drawings, in which:
**FIG. 1** illustrates a functional block diagram of an exemplary system suitable for use with the devices disclosed herein.
**FIG. 2** is a cross-sectional view of one embodiment of an occlusion sensor.
**FIG. 3** is a cross sectional view of a second embodiment of the occlusion sensor.
**FIGS. 4****(a-b)** show various embodiments of the described subject matter showing the pressure sensor located in different portions of the fluid delivery path.
**FIGS. 5****(a-b)** show exemplary graphs of voltage versus time for normal operation and operation during a partial occlusion, showing a difference in voltage across the sensor when different pressures are sensed.

### DETAILED DESCRIPTION

This disclosure presents various systems, devices, and methods for moving a liquid drug from a liquid reservoir in a drug delivery device to a patient interface, such as a needle and/or cannula. The embodiments described herein provide one or more advantages over conventional, prior art systems, components, and methods, namely, systems, devices, and methods for identifying whether an occlusion has formed in the fluid path, or even the extent to which an occlusion has formed.

Various embodiments of the presently described subject matter include systems and methods for delivering a medication to a user using a drug delivery device (sometimes referred to herein as a "pod"), either autonomously, or in accordance with a wireless signal received from an electronic device. The medication can include any drug in liquid form capable of being administered by a drug delivery device via a subcutaneous needle and/or cannula, including, for example, insulin, GLP-1, pramlintide, morphine, blood pressure medicines, chemotherapy drugs, fertility drugs, arthritis drugs, or the like, or co-formulations of two or more of GLP-1, pramlintide, and insulin. In various embodiments, the electronic device may be a user device comprising a smartphone, a smart watch, a smart necklace, a module attached to the drug delivery device, or any other type or sort of electronic device that may be carried by the user or worn on the body of the user and that executes an algorithm that computes the times and dosages of delivery of the medication.

For example, the user device may execute an "artificial-pancreas" (AP) algorithm that computes the times and dosages of delivery of a medication such as insulin. The user device may also be in communication with a sensor, such as a glucose sensor or a continuous glucose monitor (CGM), that collects data on a physical attribute or condition of the user, such as a glucose level. The sensor may be disposed in or on the body of the user and may be part of the drug delivery device or may be a separate device.

Alternatively, the drug delivery device may be in communication with the sensor in lieu of or in addition to the communication between the sensor and the user device. The communication may be direct (if, e.g., the sensor is integrated with or otherwise a part of the drug delivery device) or remote/wireless (if, e.g., the sensor is disposed in a different housing than the drug delivery device). In these embodiments, the drug delivery device contains computing hardware (e.g., a processor, memory, firmware, etc.) that executes some or all of the algorithm that computes the times and dosages of delivery of the medication.

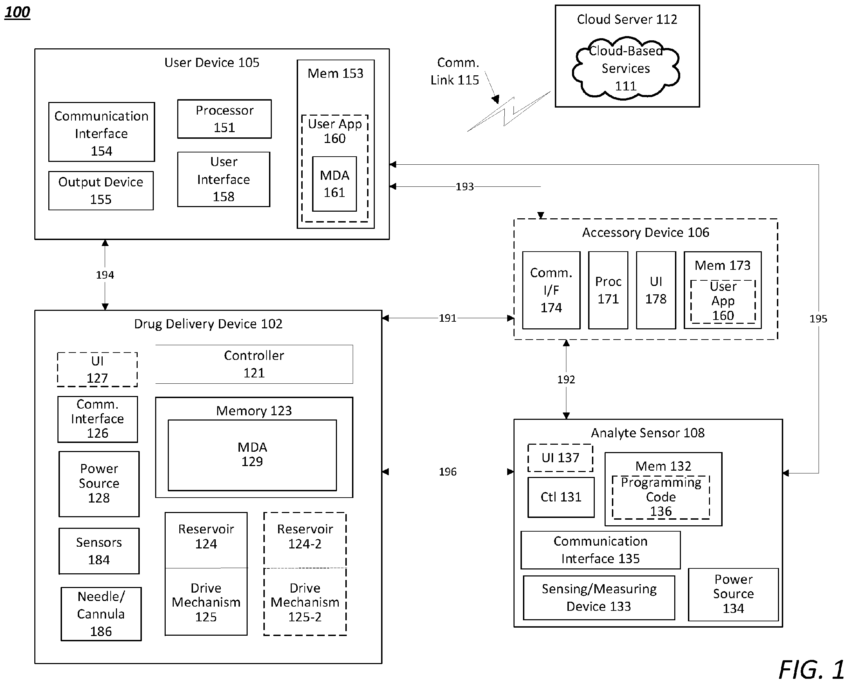

**FIG.** 1 illustrates a functional block diagram of an exemplary drug delivery system 100 suitable for implementing the systems and methods described herein. The drug delivery system 100 may implement (and/or provide functionality for) a medication delivery algorithm, such as an artificial pancreas (AP) application, to govern or control the automated delivery of a drug or medication, such as insulin, to a user (e.g., to maintain euglycemia - a normal level of glucose in the blood). The drug delivery system 100 may be an automated drug delivery system that may include a drug delivery device 102 (which may be wearable or attached directly to the skin of a user), an analyte sensor 108 (which may also be wearable), and a user device 105.

Drug delivery system 100, in an optional example, may also include an accessory device 106, such as a smartwatch, a personal assistant device, a smart insulin pen, or the like, which may communicate with the other components of system 100 via either a wired or wireless communication links 191-193.

### User Device

The user device 105 may be a computing device such as a smartphone, a smartwatch, a tablet, a personal diabetes management (PDM) device, a dedicated diabetes therapy management device, or the like. In an example, user device 105 may include a processor 151, device memory 153, a user interface 158, and a communication interface 154. The user device 105 may also contain analog and/or digital circuitry that may be implemented as a processor 151 for executing processes based on programming code stored in device memory 153, such as user application 160 incorporating medication delivery algorithm (MDA) 161 to manage a user's blood glucose levels and for controlling the delivery of the drug, medication, or therapeutic agent to the user, as well for providing other functions, such as calculating a carbohydrate-compensation dosage, a correction bolus dosage and the like as discussed below. For example, the processor 151 may be operable to execute the programming code including the MDA 161 which causes the processor to perform different functions such as those described herein. The user device 105 may be used to activate, deactivate, trigger a needle/canula insertion, program, adjust settings, and/or control operation of drug delivery device 102 and/or the analyte sensor 103 as well as the optional smart accessory device 106.

The processor 151 may also be configured to execute programming code stored in device memory 153, such as the user app 160. The user app 160 may be a computer application that is operable to deliver a drug based on information received from the analyte sensor 103, the cloud-based services 111 and/or the user device 105 or optional accessory device 106. The memory 153 may also store programming code to, for example, operate the user interface 158 (e.g., a touchscreen device, a camera or the like), the communication interface 154 and the like. The processor 151, when executing user app 160, may be configured to implement indications and notifications related to meal ingestion, blood glucose measurements, detection of an occlusion, and the like. The user interface 158 may be under the control of the processor 151 and be configured to present a graphical user interface that enables the input of a meal announcement, presentation of a notification of detection of an occlusion, adjust setting selections and the like as described herein.

In a specific example, when the user app 160 includes MDA 161, the processor 151 is also configured to execute a diabetes treatment plan (which may be stored in a memory) that is managed by user app 160. In addition to the functions mentioned above, when user app 160 is an AP application, it may further provide functionality to determine a carbohydrate-compensation dosage, a correction bolus dosage and determine a real-time basal dosage according to a diabetes treatment plan. In addition, as an MDA 161, user app 160 provides functionality to output signals to the drug delivery device 102 via communications interface 154 to deliver the determined bolus and/or basal dosages.

The communication interface 154 may include one or more transceivers that operate according to one or more radio-frequency protocols. In one embodiment, the transceivers may comprise a cellular transceiver and a Bluetooth^{®} transceiver. The communication interface 154 may be configured to receive and transmit signals containing information usable by user app 160.

User device 105 may be further provided with one or more output devices 155 which may be, for example, a speaker or a vibration transducer, to provide various signals to the user.

### Drug Delivery Device

In various exemplary embodiments, drug delivery device 102 may include a reservoir 124 and drive mechanism 125, which are controllable by controller 121, executing a medication delivery algorithm (MDA) 129 stored in memory 123, which may perform some or all of the functions of the AP application described above, such that user device 105 may be unnecessary for drug delivery device 102 to carry out drug delivery and control. For example, the controller 121 may be operable to execute the programming code including the MDA 129, which causes the controller to perform different functions such as those described herein. Alternatively, controller 121 may act to control reservoir 124 and drive mechanism 125 based on signals received from user app 160 executing on a user device 105 and communicated to drug delivery device 102 via communication link 194. Drive mechanism 125 may operate to longitudinally translate a plunger through the reservoir 124, so as to force the liquid drug through an outlet fluid port to needle / cannula 186. Alternatively, other types of drive mechanisms may be used.

In an alternate embodiment, drug delivery device 102 may also include an optional second reservoir 124-2 and second drive mechanism 125-2 which enables the independent delivery of two different liquid drugs. As an example, reservoir 124 may be filled with insulin, while reservoir 124-2 may be filled with glucagon, or pramlintide, or GLP-1, for example. In some embodiments, each of reservoirs 124, 124-2 may be configured with a separate drive mechanism 125, 125-2, respectively, which may be separately controllable by controller 121 under the direction of MDA 129. Both reservoirs 124, 124-2 may be connected to a common needle / cannula 186.

Drug delivery device 102 may be optionally configured with a user interface 127 providing a means for receiving input from the user and a means for outputting information to the user. User interface 127 may include, for example, light-emitting diodes, buttons on a housing of drug delivery device 102, a sound transducer, a micro-display, a microphone, an accelerometer for detecting motions of the device or user gestures (e.g., tapping on a housing of the device) or any other type of interface device that is configured to allow a user to enter information and/or allow drug delivery device 102 to output information for presentation to the user (e.g., alarm signals or the like).

Drug delivery device 102 includes a patient interface 186 for interfacing with the user to deliver the liquid drug. Patient interface may be, for example, a needle or cannula for delivering the drug into the body of the user (which may be done subcutaneously, intraperitoneally, or intravenously). Drug delivery device 102 may further include a mechanism for inserting the needle / cannula 186 into the body of the user, which may be integral with or attachable to drug delivery device 102. The insertion mechanism may comprise, in one embodiment, an actuator that inserts the needle / cannula 186 under the skin of the user and thereafter retracts the needle, leaving the cannula in place. The actuator may be triggered by user device 105 or may be a manual firing mechanism comprising springs or other energy storing mechanism, which causes the needle / cannula 186 to penetrate the skin of the user.

In one embodiment, drug delivery device 102 includes a communication interface 126, which may be a transceiver that operates according to one or more radio-frequency protocols, such as Bluetooth^{®}, Wi-Fi, near-field communication, cellular, or the like. The controller 121 may, for example, communicate with user device 105 and an analyte sensor 108 via the communication interface 126.

In some embodiments, drug delivery device 102 may be provided with one or more sensors 184. The sensors 184 may include one or more of a pressure sensor, a power sensor, or the like that are communicatively coupled to the controller 121 and provide various signals. For example, a pressure sensor may be configured to provide an indication of the fluid pressure detected in a fluid pathway between the patient interface 186 and reservoir 124. The pressure sensor may be coupled to or integral with the actuator for inserting the patient interface 186 into the user. In an example, the controller 121 may be operable to determine a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount. In one embodiment, analyte sensor 108 may be integral with drug delivery device 102.

Drug delivery device 102 further includes a power source 128, such as a battery, a piezoelectric device, an energy harvesting device, or the like, for supplying electrical power to controller 121, memory 123, drive mechanisms 125 and/or other components of drug delivery device 102.

Drug delivery device 102 may be configured to perform and execute processes required to deliver doses of the medication to the user without input from the user device 105 or the optional accessory device 106. As explained in more detail, MDA 129 may be operable, for example, to determine an amount of insulin to be delivered, IOB, insulin remaining, and the like and to cause controller 121 to activate drive mechanism 125 to deliver the medication from reservoir 124. MDA 129 may take as input data received from the analyte sensor 108 or from user app 160.

The reservoirs 124, 124-2 may be configured to store drugs, medications, or therapeutic agents suitable for automated delivery, such as insulin, Pramlintide, GLP-1, co-formulations of insulin and GLP-1, glucagon, morphine, blood pressure medicines, arthritis drugs, chemotherapy drugs, fertility drugs, hormonal drugs, or the like.

Drug delivery device 102 may be a wearable device and may be attached to the body of a user, such as a patient or diabetic, at an attachment location and may deliver any therapeutic agent mentioned above, to a user at or around the attachment location. A surface of drug delivery device 102 may include an adhesive to facilitate attachment to the skin of a user.

When configured to communicate with an external device, such as the user device 105 or the analyte sensor 108, drug delivery device 102 may receive signals over the wired or wireless link 194 from the user device 105 or from the analyte sensor 108. The controller 121 of drug delivery device 102 may receive and process the signals from the respective external devices as well as implementing delivery of a drug to the user according to a diabetes treatment plan or other drug delivery regimen.

### Accessory Device

Optional accessory device 106 may be, a wearable smart device, for example, a smart watch (e.g., an Apple Watch^{®}), smart eyeglasses, smart jewelry, a global positioning system-enabled wearable, a wearable fitness device, smart clothing, or the like. Accessory device 106 may alternatively be a smart insulin pen that works with drug delivery device 102 in managing blood glucose and treating diabetes of a user. Similar to user device 105, the accessory device 106 may also be configured to perform various functions including controlling or communicating with drug delivery device 102. For example, the accessory device 106 may include a communication interface 174, a processor 171, a user interface 178 and a memory 173. The user interface 178 may be a graphical user interface presented on a touchscreen display of the smart accessory device 107. The memory 173 may store programming code to operate different functions of the smart accessory device 107 as well as an instance of the user app 160, or a pared-down version of user app 160 with reduced functionality. In some instances, accessory device 107 may also include sensors of various types.

### Analyte Sensor

The analyte sensor 108 may include a controller 131, a memory 132, a sensing/measuring device 133, an optional user interface 137, a power source/energy harvesting circuitry 134, and a communication interface 135. The analyte sensor 108 may be communicatively coupled to the processor 151 of the management device 105 or controller 121 of drug delivery device 102. The memory 132 may be configured to store information and programming code 136.

The analyte sensor 108 may be configured to detect one or multiple different analytes, such as glucose, lactate, ketones, uric acid, sodium, potassium, alcohol levels or the like, and output results of the detections, such as measurement values or the like. The analyte sensor 108 may, in an exemplary embodiment, be configured as a continuous glucose monitor (CGM) to measure a blood glucose values at a predetermined time interval, such as every 5 minutes, every 1 minute, or the like. The communication interface 135 of analyte sensor 108 may have circuitry that operates as a transceiver for communicating the measured blood glucose values to the user device 105 over a wireless link 195 or with drug delivery device 102 over the wireless communication link 108. While referred to herein as an analyte sensor 108, the sensing/measuring device 133 of the analyte sensor 108 may include one or more additional sensing elements, such as a glucose measurement element, a heart rate monitor, a pressure sensor, or the like. The controller 131 may include discrete, specialized logic and/or components, an application-specific integrated circuit, a microcontroller, or processor that executes programming instructions, firmware, programming instructions stored in memory (such as memory 132), or any combination thereof.

Similar to the controller 121 of drug delivery device 102, the controller 131 of the analyte sensor 108 may be operable to perform many functions. For example, the controller 131 may be configured by programming code 136 to manage the collection and analysis of data detected by the sensing and measuring device 133.

Although the analyte sensor 108 is depicted in **FIG. 1** as separate from drug delivery device 102, in various embodiments, the analyte sensor 108 and drug delivery device 102 may be incorporated into the same unit. That is, in various examples, the analyte sensor 108 may be a part of and integral with drug delivery device 102 and contained within the same housing as drug delivery device 102 or an attachable housing thereto. In such an example configuration, the controller 121 may be able to implement the functions required for the proper delivery of the medication alone without any external inputs from user device 105, the cloud-based services 111, another sensor (not shown), the optional accessory device 106, or the like.

### Cloud-Based Services

Drug delivery system 100 may communicate with or receive services from a cloud server 122 providing cloud-based services 111. Services provided by cloud server 112 may include data storage that stores personal or anonymized data, such as blood glucose measurement values, historical IOB or TDI, prior carbohydrate-compensation dosage, and other forms of data. In addition, the cloud-based services 111 may process anonymized data from multiple users to provide generalized information related to TDI, insulin sensitivity, IOB and the like. The communication link 115 that couples the cloud server 112 to other components of system 100, for example, devices 102, 105, 106, 108 of system 100 may be a cellular link, a Wi-Fi link, a Bluetooth^{®} link, or a combination thereof.

### Communication Links

The wireless communication links 115 and 191-196 may be any type of wireless link operating using known wireless communication standards or proprietary standards. As an example, the wireless communication links 191-196 may provide communication links based on Bluetooth^{®}, Zigbee^{®}, Wi-Fi, a near-field communication standard, a cellular standard, or any other wireless protocol via the respective communication interfaces 126, 135, 154 and 174.

### Operational Example

In an operational example, user application 160 implements a graphical user interface that is the primary interface with the user and is used to activate drug delivery device 102, trigger a needle/cannula insertion, start and stop drug delivery device 102, program basal and bolus calculator settings for manual mode as well as program settings specific for automated mode (hybrid closed-loop or closed-loop).

User app 160, provides a graphical user interface 158 that allows for the use of large text, graphics, and on-screen instructions to prompt the user through the set-up processes and the use of system 100. It may also be used to program the user's custom basal insulin delivery profile, accept a recommended basal insulin delivery profile, check the status of drug delivery device 102, initiate bolus doses of insulin, make changes to a patient's insulin delivery profile, handle system alerts and alarms, or allow the user to switch between automated mode and manual mode.

User app 160 may be configured to operate in a manual mode in which user app 160 may deliver insulin at programmed basal rates and user-defined bolus amounts with the option to set temporary basal profiles. The controller 121 may also have the ability to function as a sensor-augmented pump in manual mode, using sensor glucose data provided by the analyte sensor 108 to populate the bolus calculator.

User app 160 may be configured to operate in an automated mode in which user app 160 supports the use of one or multiple target blood glucose values that may be adjusted manually or automatically by the system. For example, in one embodiment, target blood glucose values can range from 110-150 mg/dL, in 10 mg/dL increments, in 5 mg/dL increments, or other increments, but preferably 10 mg/dL increments. The experience for the user may reflect current setup flows whereby the healthcare provider assists the user to program basal rates, glucose targets and bolus calculator settings. These in turn may inform the user app 160 for insulin dosing parameters. The insulin dosing parameters may be adapted over time based on the total daily insulin (TDI) delivered during each use of drug delivery device 102. A temporary hypoglycemia protection mode may be implemented by the user for various time durations in automated mode. With hypoglycemia protection mode, the algorithm reduces insulin delivery and is intended for use over temporary durations when insulin sensitivity is expected to be higher, such as during exercise or fasting.

The user app 160 (or MDA 129) may provide periodic insulin micro-boluses based upon past glucose measurements and/or a predicted glucose over a prediction horizon (e.g., 60 minutes). Optimal post-prandial control may require the user to give meal boluses in the same manner as current pump therapy, but normal operation of the user app 160 may compensate for missed meal boluses and mitigate prolonged hyperglycemia. The user app 160 uses a control-to-target strategy that attempts to achieve and maintain a set target glucose value, thereby reducing the duration of prolonged hyperglycemia and hypoglycemia.

In some embodiments, user device 105 and the analyte sensor 108 may not communicate directly with one another. Instead, data (e.g., blood glucose readings) from analyte sensor may be communicated to drug delivery device 102 via link 196 and then relayed to user device 105 via link 194. In some embodiments, to enable communication between analyte sensor 108 and user device 105, the serial number of the analyte sensor must be entered into user app 160.

User app 160 may provide the ability to calculate a suggested bolus dose through the use of a bolus calculator. The bolus calculator is provided as a convenience to the user to aid in determining the suggested bolus dose based on ingested carbohydrates, most-recent blood glucose readings (or a blood glucose reading if using fingerstick), programmable correction factor, insulin to carbohydrate ratio, target glucose value, and insulin on board (IOB). The user's IOB may be estimated by user app 160 considering any manual bolus and insulin delivered by the algorithm.

### Description of Embodiments

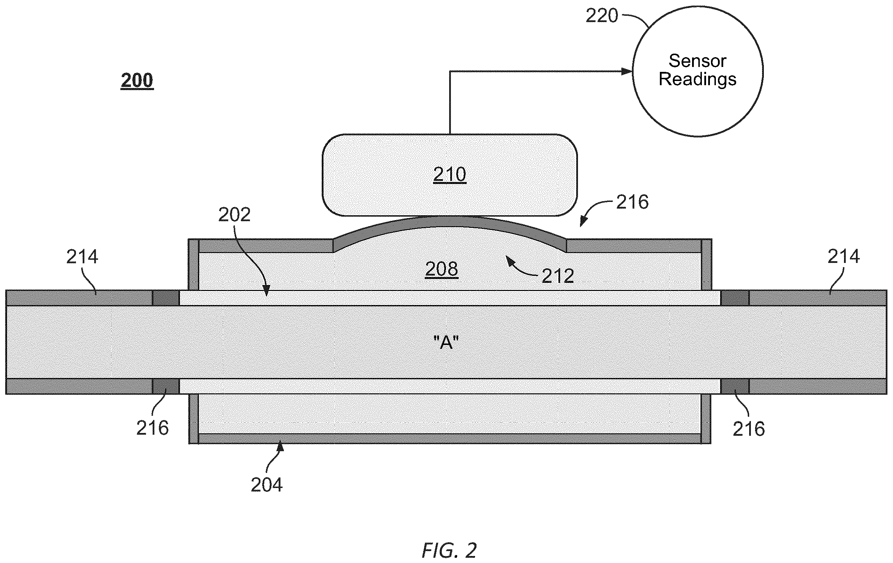

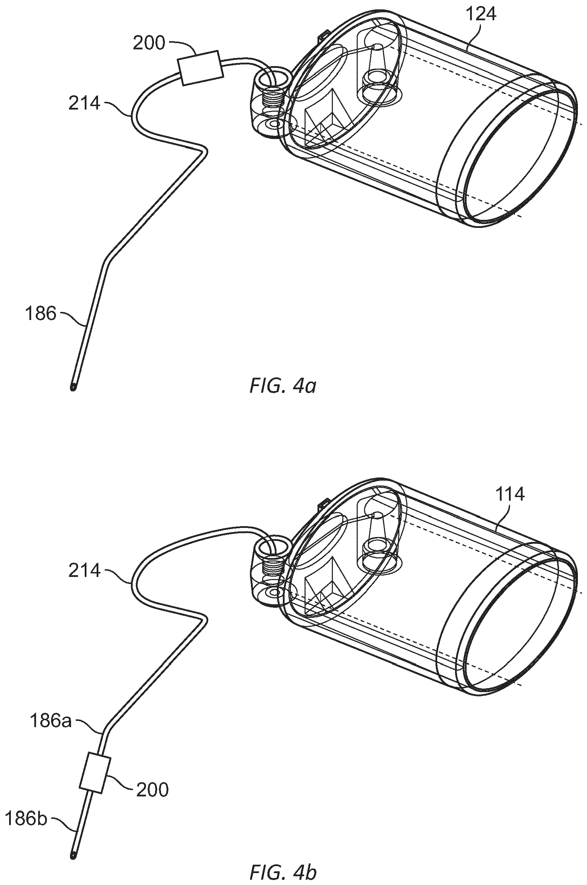

A first aspect of the described subject matter is shown in **FIG. 2****,** showing a cross-sectional view of an exemplary embodiment of the pressure sensor 200. When deployed, the pressure sensor 200 is disposed in-line with fluid path 214, at any point along the fluid path between reservoir 124 and the end of needle 186. **FIG. 4** shows two exemplary arrangements. In **FIG. 4a****,** sensor 200 is disposed in a flexible portion 212 of fluid path 214 between reservoir 124 and needle 186. An alternate embodiment is shown in **FIG. 4b** wherein sensor 200 has both distal and proximal ends coupled to portions of needle 186a, 186b. In the embodiment shown, pressure sensor 200 is bidirectional, wherein the liquid drug "A" could flow in either direction. In one embodiment, pressure sensor 200 may be housed within a housing of drug delivery device 102. In other embodiments, pressure sensor 200 may be external to the housing of drug delivery device 102.

Pressure sensor 200 includes a length of soft tubing 202, coupled at either end to fluid path 214 via couplers 216. In an example, the soft tubing 202 is more compliant than the tubing used for fluid path 214 such that a pressure buildup in fluid path 214 caused by an occlusion causes the soft tubing 202 to expand before or substantially more than other portions of fluid path 214 expand. In some embodiments, the soft tubing may be composed of a flexible polymer, in particular an elastomer. In some embodiments, the soft tubing 202 has a Young's modulus of less than 10 GPa, more specifically less than 5 GPa and in particular less than 1 GPa. In some embodiments, the soft tubing 202 has a Young's modulus between about 0.01 MPa to about 1 GPa, more specifically between about 0.1 MPa to about 100 MPa and in particular between about 0.5 MPa to about 10 MPa. In some embodiments, the fluid path 214 has a Young's modulus of at least 10 GPa, more specifically at least 30 GPa and in particular at least 60 GPa. In some embodiments, the fluid path 214 has a Young's modulus between about 10 GPa to about 1000 GPa, more specifically between about 30 GPa to about 500 GPa and in particular between about 60 GPa to about 200 GPa. For example, if fluid path 214 is made up of a hard walled needle, soft tubing 202 would experience all or nearly all of the flexion due to the pressure buildup, as the material is more compliant. Soft tubing 202 may be composed of, for example, polyvinylchloride or the like.

Soft tubing 202 is surrounded by a rigid, non-compliant enclosure 204 containing a non-compressible, or relatively non-compressible, inert fluid, such as, for example, water. In an example, fluid 208 is biocompatible and inert with respect to its effects on polymers and metals, and with respect to the body of a patient, in the event the inert fluid were to ever leak into fluid path 214. Rigid enclosure 204 may have any cross-sectional shape, for example, circular, square, oval, etc. Preferably, one or more seals (not visible) may be provided where the soft tubing 202 passes through the rigid enclosure 204 so as to seal the fluid 208 within rigid enclosure 204. Rigid, non-compliant enclosure 204 may be composed of, for example, a rigid polymer.

Rigid enclosure 204 may be configured with, or comprise, a flexible, fluid-tight region 206. In an example, the flexing 212 of flexible region 206 is caused by an increase in pressure in the fluid path and is sensed by sensor 210. The sensing may be, for example, resistive, conductive, magnetic, physical, etc. Flexible region 206 may be composed of, for example, a flexible polymer, e.g. an elastomer, or the like. In some embodiments, the flexible, fluid-tight region 206 has a Young's modulus of less than 10 GPa, more specifically less than 5 GPa and in particular less than 1 GPa. In some embodiments, the flexible, fluid-tight region 206 has a Young's modulus between about 0.01 MPa to about 1 GPa, more specifically between about 0.1 MPa to about 100 MPa and in particular between about 0.5 MPa to about 10 MPa. In some embodiments, the rigid enclosure 204 has a Young's modulus of at least 10 GPa, more specifically at least 30 GPa and in particular at least 60 GPa. In some embodiments, the rigid enclosure 204 has a Young's modulus between about 10 GPa to about 1000 GPa, more specifically between about 30 GPa to about 500 GPa and in particular between about 60 GPa to about 200 GPa.

In one exemplary embodiment, flexible region 206 may comprise a strain gauge comprising a polymer substrate having conductive inks coated thereon wherein the (electrical) resistance of the inks changes as the shape of flexible region 206 changes. In such an embodiment, sensor 210 measures the changes in (electrical) resistance as flexible region 206 flexes. In another exemplary embodiment, flexible region may comprise an optically reflective surface and sensor 210 may be, for example, an optical emitter/detector pair which detects changes in the reflectivity of flexible region 206 as it flexes. Changes in reflectivity may correspond for example to the intensity, angle or wavelength of the reflected light. In yet another exemplary embodiment, sensor 210 may be a micro-electromechanical system (MEMS) component having a physical contact with the flexible region 206. In yet another exemplary embodiment, flexible region 206 is configured with a magnetic region and the movement of the magnetic field produced by the magnetic region as flexible region 206 flexes is sensed by a sensor, for example, a Hall effect sensor. It should be noted that the described subject matter is not meant to be limited to embodiments using the specific sensors or means of sensing mentioned herein. Any sensor 210 capable of detecting a change in the shape of flexible region 206 may be used.

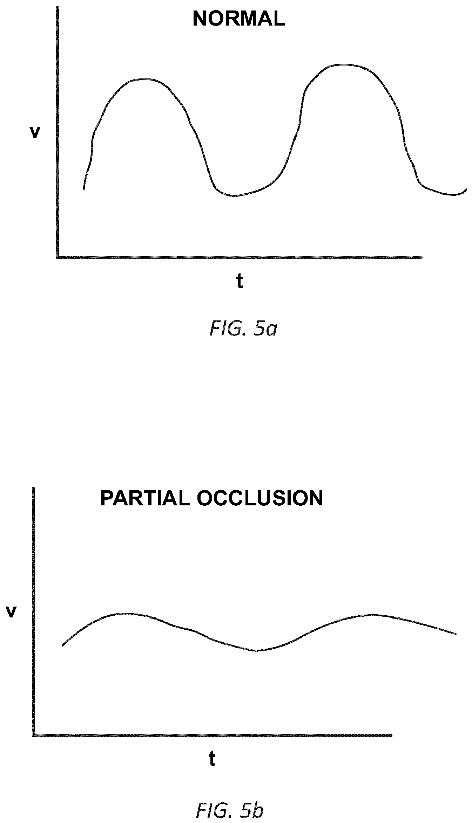

In an operational example, a full or partial occlusion may cause an increase in pressure within fluid path 214 and, in turn within the soft tubing 202. Because soft tubing 202 is more compliant than the rest of fluid path 214, soft tubing 202 expands since the more compliant soft tubing 202 experiences a deformation due to the change in pressure to a greater extent than other portions of fluid path 214. The incompressible fluid 208 disposed within rigid container 204 transfers the expansion of soft tubing 202 to flexible region 206, causing flexible region 206 to flex outward 212 from rigid enclosure 204. Sensor 210 detects the outward flexing of flexible region 206 by any of the means mentioned, or by any other means, and produces pressure readings 220. Over time, pressure readings 220 may form a pressure profile (e.g., indicating pressure values that occur during normal operation of the pump) of the fluid path that can be analyzed to determine the existence, extent, and type of the occlusion. In addition to pressure profile(s) that depict normal operation of the pump, as shown in **FIG. 5a****,** pressure profile(s) for occlusion(s), as shown in **FIG. 5b****,** may also be generated. In FIGs. **5a** and **5b****,** the vertical axis represents pressure values (v) with a zero value at the origin and the horizontal axis represents time. The pressure profiles of normal operation and the pressure profiles of occlusion may be stored in a memory, such as those described with reference to **FIG. 1****.** As pressure sensor readings 220 are generated, such sensor readings may be compared with such pressure profiles to determine whether the pump is operating normally or whether an occlusion has developed (or is in the process of developing), as explained in further detail below.

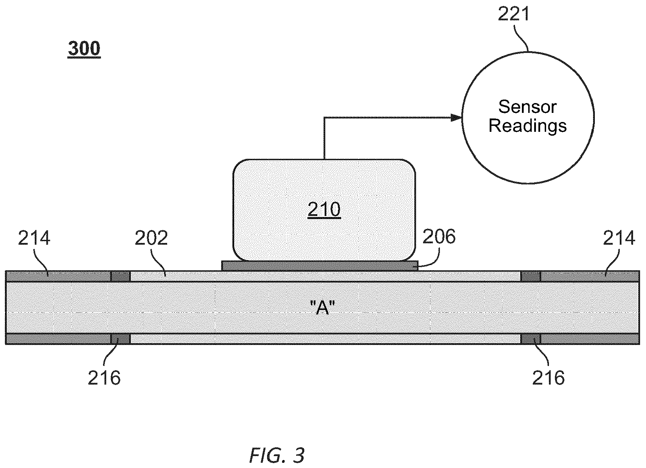

In another embodiment 300, shown in **FIG. 3****,** flexible region 206 may be disposed directly on soft tubing 202, so that when soft tubing 202 expands, flexible region 206 also expand and sensor 210 detects the outward flexing of flexible region 206 by any of the examples mentioned above, in this example, eliminates the need for rigid, non-compliant enclosure 204. The sensor readings 221 may be different from or the same as the sensor readings 220 generated in the example of FIG. 2.

In this example of the described subject matter, the sensor readings 221 may be input to an algorithm, or machine learning model trained, to detect and classify occlusions based on readings from the sensor 210. Different types of occlusions may produce different pressure profiles. For example, for each pulse produced by the pumping mechanism, an increase in pressure may be realized within fluid path 214. If a partial occlusion exists, an increase in pressure due to a particular pulse of the pump may plateau and decay over time, until the next pulse from the pumping mechanism. If a full occlusion exists, the increase in pressure may plateau and remain constant and may increase during the next pulse from the pumping mechanism. In one embodiment, an output of the pressure sensor 210 may be voltage. For example, the pressure detector may be implemented as a strain gauge or a pressure detector that outputs, or causes output of, a voltage. As the pump operates and the fluid line experiences increases and decreases in pressure, like a pulse, the strain gauge may be operable to change in (electrical) resistance and the voltage detected by the sensor may change. For example, a higher pressure may result in a lower (electrical) resistance across sensor 200, resulting in a higher voltage. **FIG. 5a** shows an exemplary graph of time versus voltage for normal operation. In this case, low pressure detected by detector 200 results in a low (electrical) resistance and therefore a high voltage. **FIG. 5b** shows an exemplary graph of time versus voltage when a partial occlusion occurs. During this time, the pressure sensed by sensor 200 increases, resulting in an increase in (electrical) resistance and a corresponding drop in voltage. In another example, a higher pressure may result in a higher (electrical) resistance across sensor 210, resulting in a higher voltage. Accordingly, when there is no occlusion, a low pressure may be detected by the detector which is increased when the pump is pumping and subsequently returns to the low pressure detected before the pumping. When there is a full occlusion, a low pressure may be detected by the detector prior to pumping, followed by an increased pressure during and after pumping, which does not return to the low pressure detected prior to pumping. When there is a partial occlusion, a low pressure may be detected by the detector prior to pumping, followed by an increased pressure during and after pumping, which returns slowly (slower than when there is no occlusion) to the low pressure detected before the pumping.

The pressure profiles can be used by a processor, such as those described with reference to FIG. 1, to determine or to inform a determination that a full or partial occlusion to the fluid path 214 is present. A pressure profile showing normal (i.e., occlusion-less) pressures detected by sensor 210 can be stored in memory and can be compared with a pressure profile collected during an occlusion, with the difference between the pressure profiles indicating a possible occlusion. For example, when a substantial aberration in the current pressure profile occurs, this may be indicative of the presence of an inclusion. The pressure profiles may be continuously generated based on signals from the sensor 210 as the liquid drug is delivered to the user and can be constantly compared with pressure profiles of normal operation. The pressure profiles for normal operation can be collected and stored in a memory for a particular user, or as an average over a population of users, which may be stored by a cloud-based service or the like.

In another aspect of the described subject matter, the machine learning model may be further trained on an input of glucose readings produced by a glucose sensor 108. The machine learning model may use the glucose readings to confirm or increase the probability of the detection and classification of the readings from sensor 210 as an occlusion. Any occlusion may cause the amount of the liquid drug (e.g., insulin) delivered to the patient to be reduced which, in the case in which the liquid drug is insulin, may cause an unexpected increase in the user's blood glucose level. The machine learning model may also be trained on historic profiles of the user's blood glucose readings and the user's blood glucose readings response to pumping or expelling various quantities of insulin. Deviations from historic profiles of the user's blood glucose readings, given the same amount of insulin being pumped, may be determined by the processor to indicate that not all of the insulin is being delivered to the user, thereby confirming, or increasing the probability of the detection of an occlusion.

The machine learning model may associate pressure profiles detected in the fluid path with normal dosing patterns and may apply multivariate techniques to identify an occlusion. Examples of the multivariate techniques may include multiple regression analysis, logistic regression analysis, discriminant analysis, multivariate analysis of variance, factor analysis, cluster analysis, multidimensional scaling, correspondence analysis, conjoint or trade-off analysis, canonical correlation, structural equation modeling, or the like.

In some instances, a machine learning model may be implemented as part of medication delivery algorithm 129 or 161 and may inform medication delivery algorithm 121 or 161 of the need to notify the user of the occlusion. A reduction in the amount of insulin delivered to the patient may result in a dangerous situation for the patient. For example, the patient may become hyperglycemic if the occlusion is allowed to persist or is uncompensated for.

In some instances, a threshold may be established for determining when to notify the user. In an embodiment, the threshold may be a pressure threshold that, when exceeded, as sensed by the occlusion sensor, the user is notified. The pressure threshold may depend upon the type of drug delivery device 102 used, for example based on the type of pump used. In another embodiment, a reduction of the volume of the insulin delivered, for example, by a present amount, such as approximately 3 Units, or approximately 30 µL, approximately 5 units, or the like, may be used as a threshold for notifying the user of the occlusion. In another embodiments, a reduction of the volume of the insulin delivered by at least more than 2%, more specifically at least more than 5% and in particular at least 10% may be used as a threshold for notifying the user of the occlusion. In some instances, the medication delivery algorithm may attempt to compensate for partial occlusion by pumping more insulin or may attempt to clear an occlusion by increasing the pressure or pumping additional pulses (which correspond to, for example, a further translation of a plunger within a reservoir, or a further cycle of the pump). In the event that medication delivery algorithm 121 or 161 is not able to compensate for the occlusion, a recommendation may be presented, such as on user interface 158 of user device 105 of FIG. 1, to the user to switch out the drug delivery device 102 with a new drug delivery device.

As would be realized by one of skill in the art, many variations on the embodiments and examples disclosed herein are possible. In particular, assorted sizes, materials and configurations are contemplated to be the within the scope of the described subject matter and the described subject matter is not meant to be limited by the specific embodiments disclosed herein.

The following examples pertain to various embodiments disclosed herein for the needle insertion/reduction mechanism for use with an automated drug delivery system.

Example 1 is a device for detecting changes in pressure in a fluid path comprising a length of tubing connected in line with the fluid path, a rigid enclosure surrounding the tubing, a flexible region defined in a wall of the rigid enclosure, a fluid disposed in the rigid structure and a sensor capable of detecting flex of the flexible region defined in the wall of the rigid enclosure.

Example 2 is an extension of Example 1, or any other example disclosed herein, wherein the length of tubing is constructed of a material more compliant in the material of which the rest of the fluid path is constructed such that a bill of a pressure within the fluid path caused by causes the length of tubing to expand, wherein expansion of the length of tubing is communicated to the flexible region by the fluid.

Example 3 is an extension of Example 1, or any other example disclosed herein, wherein the flexible region comprises a strain gauge wherein the (electrical) resistance of the strain gauge changes as a flexible region flexes and further wherein the sensor senses changes in the (electrical) resistance.

Example 4 is an extension of Example 1, or any other example disclosed herein, wherein the flexible region comprises a reflective optical service and further wherein the sensor comprises a light emitter/detector pair which detects changes in the reflectivity of the surface of the flexible region as a flexible region expands.

Example 5 is an extension of Example 1, or any other example disclosed herein, wherein the flexible region comprises a magnetic surface or magnetic region and further wherein movement of the of a magnetic field generated by the magnetic surface or magnetic region is detected by the sensor.

Example 6 is an extension of Example 1, or any other example disclosed herein, wherein the sensor is a MEMS device physically coupled to the flexible region.

Example 7 is an extension of example 1, or any other example disclosed herein, wherein the sensor outputs a time series of readings indicative of the change in pressure within the fluid path.

Example 8 is an extension of Example 7, or any other example disclosed herein, wherein the time series of readings forms a pressure profile of the pressure within the fluid path

Example 9 is an extension of Example 8, or any other example disclosed herein, wherein the time series of readings as input to a machine learning model trained to detect and classify occlusions in the fluid path.

Example 10 is an extension of Example 9, or any other example disclosed herein, wherein the occlusion is in fluid path may be full occlusion or a partial occlusion.

Example 11 is an extension of Example 9, or any other example disclosed herein, wherein the machine learning model is also trained on historic profiles indicating changes in glucose readings of a user based on a given amount of insulin being delivered.

Example 12 is an extension of Example 11, or any other example disclosed herein, wherein the machine learning model also takes as input blood glucose readings and further wherein the blood glucose readings can increase the confidence in the detection and classification of the occlusion.

Example 13 is an extension of Example 12, or any other example disclosed herein, wherein a user is informed of the occlusion based on a reduction in the volume of liquid drug delivered to the user.

Example 14 is an extension of Example 13, or any other example disclosed herein, wherein the liquid drug is insulin and further wherein the user is informed when the volume of insulin delivered to the user is reduced by a preset amount, such as approximately 3 units.

Example 15 is a drug delivery device comprising: a processor, programming instructions, implementing a medication delivery algorithm executing on the processor, the programming instructions performing the functions of: receiving a time series of readings from a sensor configured to detect changes in pressure within a fluid path of the drug delivery device and inputting the time series of readings to machine learning model trained to detect and classify occlusions within the fluid path.

Example 16 is an extension of Example 15, or any other example disclosed herein, wherein the programming instructions performs the further function of determining a reduction in volume of a liquid drug delivered to a user of the drug delivery device.

Example 17 is an extension of Example 16, or any other example disclosed herein, wherein the programming instructions performs the further function of compensating for the reduction in volume of the liquid drug delivered to the user.

Example 18 is an extension of Example 16, or any other example disclosed herein, wherein the programming instructions performs the further function of attempting to clear the fluid path of the occlusion by increasing the pressure in the fluid path.

Example 19 is an extension of Example 16, or any other example disclosed herein, wherein the programming instructions performs the further function of informing the user of the occlusion when the reduction in volume of the liquid drug delivered to the user reaches a predetermined threshold.

Example 20 is extension of Example 10, or any other example disclosed herein, wherein the liquid drug is insulin and further wherein the predetermined threshold is a preset amount, such as approximately 3 units.

Example 21 is extension of Example 9, or any other example disclosed herein, wherein processor is further operable to receive blood glucose readings and utilize the blood glucose readings to determine a confidence in the detection and classification of the occlusion.

Software related implementations of the techniques described herein may include, but are not limited to, firmware, application specific programming code, programming instructions, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

To those skilled in the art to which the described subject matter relates, many modifications and adaptations of the described subject matter may be realized. Implementations provided herein, including sizes, shapes, ratings, compositions and specifications of various components or arrangements of components, and descriptions of specific manufacturing processes, should be considered exemplary only and are not meant to limit the described subject matter in any way. As one of skill in the art would realize, many variations on implementations discussed herein which fall within the scope of the described subject matter are possible. Moreover, it is to be understood that the features of the various embodiments described herein were not mutually exclusive and can exist in various combinations and permutations, even if such combinations or permutations were not made express herein, without departing from the spirit and scope of the described subject matter. Accordingly, the method and apparatus disclosed herein are not to be taken as limitations on the described subject matter but as an illustration thereof. The scope of the described subject matter is defined by the claims which follow.

The present disclosure furthermore relates to computer programs comprising instructions (also referred to as computer programming instructions) to perform the aforementioned functionalities. The instructions may be executed by a processor. The instructions may also be performed by a plurality of processors for example in a distributed computer system. The computer programs of the present disclosure may be for example preinstalled on, or downloaded to the medicament delivery device, management device, fluid delivery device, e.g. their storage. Although the aforementioned description distinguishes between a system and a device, that a device according to the claims may also be a system.

Although the present invention is defined in the attached claims, it should be understood that the present invention can also (alternatively) be defined in accordance with the following embodiments:
1. A device for detecting changes in pressure in a fluid path comprising:
   a length of tubing connected in-line with the fluid path;
      and
   a sensor operable to detect flex of the length of tubing.
2. The device of embodiment 1, wherein the sensor comprises a flexible member disposed on the length of tubing.
3. The device of embodiment 1, wherein the sensor comprises:
   a rigid enclosure surrounding the length of tubing;
   a flexible region defined in a wall of the rigid enclosure; and
   a fluid disposed in the rigid enclosure,
   wherein the sensor is operable to detect the flex of the length of tubing by detecting flex of the flexible region of the rigid enclosure.
4. The device of embodiment 3, wherein the length of tubing is constructed of a material more compliant than the material of which the rest of the fluid path is constructed such that a buildup of pressure within the fluid path caused by an occlusion in the fluid path causes the length of tubing to expand, wherein expansion of the length of tubing is communicated to the flexible region by the fluid.
5. The device of embodiment 3, wherein the flexible region comprises a strain gauge wherein a resistance of the strain gauge changes as the flexible region flexes and the sensor is operable to sense the changes in the resistance.
6. The device of embodiment 3, wherein the flexible region comprises a reflective optical surface and the sensor comprises a light emitter/detector pair which detects changes in the reflectivity of the surface of the flexible region as the flexible region expands.
7. The device of embodiment 3, wherein the flexible region comprises a magnetic surface and movement of a magnetic field generated by the magnetic surface is detected by the sensor.
8. The device of embodiment 3, wherein the sensor is a micro-electromechanical system device physically coupled to the flexible region.
9. The device of embodiment 3, wherein the sensor is configured to output a time series of readings indicative of changes in pressure within the fluid path.
10. The device of embodiment 9, further comprising:
   a processor operable to:
   receive the time series of readings from the sensor; and
   form a pressure profile of the pressure within the fluid path.
11. The device of embodiment 10, wherein the processor is further operable to:
   detect occlusions in the fluid path based on the time series of readings.
12. The device of embodiment 11, wherein the processor is further operable to:
   classify detected occlusions in the fluid path as full occlusions or partial occlusions.
13. The device of embodiment 11, wherein the processor is further operable to:
   use on historical profiles indicating changes in glucose readings based on a given amount of insulin being delivered in the detection of the occlusions.
14. The device of embodiment 13, wherein the processor is further operable to:
   receive blood glucose readings and utilize the blood glucose readings to determine a confidence in the detection and classification of the occlusion.
15. The device of embodiment 14, wherein the processor is further operable to:
   generate a notification of the detected occlusion.
16. The device of embodiment 15, wherein the liquid drug is insulin and the processor is further operable to:
   generate notification when the volume of insulin delivered to the user is reduced by a preset amount.
17. A drug delivery device, comprising:
   a processor; and
   a memory storing programming instructions, implementing a medication delivery algorithm executable by the processor, the programming instructions when executed by the processor cause the processor to:
      receive a time series of readings from a sensor configured to detect changes in a fluid path of the drug delivery device; and
      utilize the time series of readings to detect and classify occlusions within the fluid path.
18. The drug delivery device of embodiment 17, wherein the programming instructions when executed by the processor further cause the processor to:
   determine a reduction in volume of a liquid drug output by the drug delivery device.
19. The drug delivery device of embodiment 17, wherein the programming instructions when executed by the processor further cause the processor to:
   compensating for the reduction in volume of the amount of liquid drug delivered to the user by causing the drug delivery device to output greater amounts of the liquid drug.
20. The drug delivery device of embodiment 17, wherein the programming instructions when executed by the processor further cause the processor to:
   determine that there is an occlusion in the fluid path; and attempt to clear the fluid path of the occlusion by causing the pump to increase pressure in the fluid path.
21. The drug delivery device of embodiment 18, wherein the programming instructions when executed by the processor further cause the processor to:
   generate a notification of an occlusion when the determined reduction in volume of liquid drug output by the drug delivery device reaches a predetermined threshold.

## Claims

1. A device for detecting changes in pressure in a fluid path comprising:
a length of tubing connected in-line with the fluid path;
and
a sensor operable to detect flex of the length of tubing.

2. The device of claim 1, wherein the sensor comprises a flexible member disposed on the length of tubing.

3. The device of claim 1 or 2, wherein the sensor comprises:
a rigid enclosure surrounding the length of tubing;
a flexible region defined in a wall of the rigid enclosure; and
a fluid disposed in the rigid enclosure,
wherein the sensor is operable to detect the flex of the length of tubing by detecting flex of the flexible region of the rigid enclosure.

4. The device of any one of claims 1 to 3, wherein the length of tubing is constructed of a material more compliant than the material of which the rest of the fluid path is constructed such that a buildup of pressure within the fluid path caused by an occlusion in the fluid path causes the length of tubing to expand, wherein expansion of the length of tubing is communicated to the flexible region by the fluid.

5. The device of claim 3 or 4, wherein the flexible region comprises a strain gauge wherein a resistance of the strain gauge changes as the flexible region flexes and the sensor is operable to sense the changes in the resistance, and/or
wherein the flexible region comprises a reflective optical surface and the sensor comprises a light emitter/detector pair which detects changes in the reflectivity of the surface of the flexible region as the flexible region expands, and/or
wherein the flexible region comprises a magnetic surface or magnetic body and movement of a magnetic field generated by the magnetic surface or body is detected by the sensor, and/or
wherein the sensor is a micro-electromechanical system device physically coupled to the flexible region.

6. The device of any preceding claim, wherein the sensor is configured to output a time series of readings indicative of changes in pressure within the fluid path.

7. The device of any one of claims 1 to 6, further comprising:
a processor operable to:
receive the time series of readings from the sensor; and
form a pressure profile of the pressure within the fluid path.

8. The device of claim 7, wherein the processor is further operable to:
detect occlusions in the fluid path based on the time series of readings,
in particular wherein the processor classifies detected occlusions in the fluid path as full occlusions or partial occlusions.

9. The device of claim 7 or 8, wherein the processor is further operable to:
use historical profiles indicating changes in glucose readings based on a given amount of insulin being delivered in the detection of the occlusions.

10. The device of any one of claims 8 to 9, wherein the processor is further operable to:
receive blood glucose readings and utilize the blood glucose readings to determine a confidence in the detection and classification of the occlusion.

11. The device of any one of claims 8 to 10, wherein the processor is further operable to:
generate a notification of the detected occlusion and/or
generate notification when the volume of insulin delivered to the user is reduced by a preset amount.

12. The device of claim 10 or 11, wherein the processor is further operable to:
attempt to clear the fluid path of the occlusion by causing the pump to increase pressure in the fluid path, when determining that there is an occlusion in the fluid path.

13. A method for detecting occlusions within a fluid path of a drug delivery device, wherein the method comprises the following steps:
receive a time series of readings from a sensor configured to detect changes in a fluid path of the drug delivery device; and
utilize the time series of readings to detect and classify occlusions within the fluid path.

14. The method of claim 13, wherein the programming instructions when executed by the processor further cause the processor to:
determine a reduction in volume of a liquid drug output by the drug delivery device.

15. The method of claim 14, wherein the programming instructions when executed by the processor further cause the processor to:
compensate for the reduction in volume of the amount of liquid drug delivered to the user by causing the drug delivery device to output greater amounts of the liquid drug, in particular greater amounts which correspond to the determined reduction in volume of a liquid drug output by the drug delivery device
and/or
generate a notification of an occlusion when the determined reduction in volume of liquid drug output by the drug delivery device reaches a predetermined threshold.
